# EUROPEAN PATENT SPECIFICATION

(11) **EP 3 038 510 B1**
(45) Date of publication and mention of the grant of the patent: **05.08.2020**
(21) Application number: 14840559.0
(22) Date of filing: 27.08.2014
(51) Int. Cl.: A61B 1/05, A61B 1/00, A61B 1/04, A61B 1/06, A61B 1/267

(54) **VISUALIZATION INSTRUMENT**
VISUALISIERUNGSINSTRUMENT
INSTRUMENT DE VISUALISATION

(30) Priority: 27.08.2013 US 201361870783 P; 26.08.2014 WO PCT/US2014/052780
(43) Date of publication of application: 06.07.2016
(73) Proprietor: King Systems Corporation, Noblesville, IN 46060 (US)
(72) Inventor: HRUSKA, Amy, Indianapolis, IN 46220 (US); WAGNER, Zachary, Noblesville, IN 46060 (US); McGRAIL, Thomas W., Cicero, IN 46034 (US); YEH, Yun Siung Tony, Libertyville, IL (US); COBURN, Caleb, Noblesville, IN 46060 (US); ROLFS, Bryan E., Chicago, IL 60618-5357 (US); WEIDLING, Paul, Noblesville, IN 46060 (US); STREICHER, Matt, Indianapolis, IN 46216 (US)
(74) Representative: AWA Denmark A/S
(86) International application number: PCT/US2014/053019
(87) International publication number: WO 2015/031543

(56) References cited:
- WO-A1-2012/097181
- US-A1- 2010 288 272
- US-A1- 2011 077 466
- US-A1- 2011 130 627
- US-A1- 2012 330 324
- US-B1- 7 946 981
- US-B1- 7 946 981
- US-B2- 6 296 893
- US-B2- 8 317 509
- US-B2- 8 366 612

## Description

### FIELD OF THE DISCLOSURE

The present disclosure relates generally to a visualization instrument including a camera communicatively coupled with a display device. More specifically, the present disclosure relates to a visualization instrument including a camera insertable into an internal space.

### BACKGROUND OF THE DISCLOSURE

Visualization instruments include medical and non-medical instruments. Medical visualization instruments are used in a multitude of procedures including laryngoscopy, colonoscopy, rhinoscopy, bronchoscopy, cystoscopy, hysteroscopy, laparoscopy, arthroscopy, etc. Generally, a medical visualization instrument comprises a camera and structure arranged to support the camera during the procedure. The structure may be configured for the particular procedure, and the instrument may thus be given a name corresponding to the procedure. Exemplary instruments include laryngoscopes, bronchoscopes, endoscopes etc. Non-medical visualization instruments are used to investigate the internal structures of machines, buildings, and explosive devices, for example.

Laryngoscopes provide views of the vocal folds and the glottis after the laryngoscope has been inserted into the buccal cavity of the patient. Direct laryngoscopy is usually carried out with the patient lying on his or her back. During direct laryngoscopy, the laryngoscope is inserted into the mouth, typically on the right side, and pushed towards the left side to move the tongue out of the line of sight and to create a pathway for insertion of an endotracheal tube. The blade may be lifted with an upward and forward motion to move the epiglottis and make a view of the glottis possible. Once the laryngoscope is in place, the endotracheal tube may be inserted into the pathway. The blade may be provided with guide surfaces to guide the insertion of the endotracheal tube.

Laryngoscopes may be outfitted with optical devices to provide views of the vocal cords externally of the patient's body. Optical devices include lenses, mirrors and fiberoptic fibers, all adapted to transfer an optical image. Devices may also be provided to capture the optical images and display corresponding images in video display screens and/or monitors. Visualization instruments, such as laryngoscopes, are known from WO2012/097181 as well as US7946981 and US2011/130627.

Traditional visualization instruments have limitations such as, for example, fogging, insufficient lighting to produce a good optical image, inability to project images remotely, additional procedural steps to insert the endotracheal tube, and cost, to name a few. Further, there is a need to reduce the size of the camera to reduce the invasiveness of medical procedures and for pediatric care.

### SUMMARY OF EMBODIMENTS OF THE DISCLOSURE

A visualization instrument and a method of making the visualization instrument are disclosed herein. In an exemplary embodiment, the visualization instrument is a video laryngoscope. In another exemplary embodiment, the visualization instrument is configured for non-medical uses. In embodiments of the visualization instrument, the visualization instrument includes a camera. The camera includes a light source configured to illuminate structures in a target space; an image sensor having an imaging surface; and an optical train including one or more lenses. The optical train is configured to receive light reflected from the illuminated structures and refract optical images of the illuminated structures to the image sensor. The image sensor generates an image stream including images corresponding to the optical images. The camera also includes a support structure supporting the light source, the image sensor and the optical train; and a housing enclosing the support structure, the light source, the image sensor and the optical train.

In embodiments of the visualization instrument, the visualization instrument comprises an image presentation component including a display screen and a battery housing; a camera harness coupled to the image presentation component, the camera harness including a wire harness and a camera, the camera including a light source configured to illuminate structures in a target space, an image sensor, and an optical train including one or more lenses, the optical train configured to receive light reflected from the illuminated structures and refract optical images of the illuminated structures to the image sensor, the image sensor generating an image stream corresponding to the optical images. The instrument further comprises a blade including a handle portion (42) integrally formed with an insertable portion, the handle portion having a proximal cavity configured to receive the battery housing, the insertable portion comprising a guide pathway adapted to guide an elongate device, and an electronics pathway formed by a blade body and an affixable wall seamed to the body portion, the electronics pathway configured to receive the camera harness when the image presentation component is inserted into the proximal cavity of the blade.

Embodiments of a method of making a low cost visualization instrument are also disclosed herein. In one example, the method comprises: providing an image presentation component including a display screen and a battery housing, a camera including a light source configured to illuminate structures in a target space, an image sensor, and an optical train including one or more lenses, the optical train configured to receive light reflected from the illuminated structures and refract optical images of the illuminated structures to the image sensor, and the image sensor generating an image stream corresponding to the optical images. The method further comprises injection molding a blade having a handle portion integrally formed with an insertable portion, the handle portion having a proximal cavity configured to receive the battery housing, the insertable portion comprising a guide pathway adapted to guide an elongate device, and an electronics pathway formed by a blade body. The method further comprises molding an affixable wall; affixing the affixable wall to the blade body portion; and forming a camera harness by molding a polymeric material around a wire bundle, the wire bundle connected to the camera and to a circuit board, wherein the electronics pathway is configured to receive the camera harness when the image presentation component is inserted into the proximal cavity of the blade.

The features of this invention, and the manner of attaining them, will become more apparent and the invention itself will be better understood by reference to the following description of embodiments of the invention taken in conjunction with the accompanying drawings. The invention is defined in independent claim 1 and the dependent claims 2 - 7. All other embodiments described in the description are for exemplary purposes only.

### BRIEF DESCRIPTION OF THE DRAWINGS

FIGS. 1 and 2 are posterior and lateral plan views of an embodiment of a video laryngoscope set forth in the disclosure;
FIG. 3 is a plan view of the distal end of the video laryngoscope of FIGS. 1 and 2;
FIG. 4 is a perspective view of an embodiment of a camera set forth in the disclosure;
FIG. 5 is an exploded view of another embodiment of a camera set forth in the disclosure;
FIGS. 6 and 7 are exploded and assembled views of an embodiment of a video laryngoscope set forth in the disclosure;
FIGS. 8 to 15 are views of embodiments of a blade adapter and components thereof set forth in the disclosure;
FIGS. 16 and 17 are exploded views of an embodiment of a camera and adapter set forth in the disclosure;
FIGS. 18 to 23 are views of embodiments of channelled and channelless blades set forth in the disclosure;
FIGS. 24 to 27 are partial views of embodiments of polymer injection molds configured to manufacture the blades of FIGS. 18 to 23 as set forth in the disclosure;
FIGS. 28 to 31 are cross-sectional views and a flowchart depicting a method set forth in the disclosure of forming a camera harness; and
FIGS. 32 to 34 are perspective views of an embodiment of a positioning device set forth in the disclosure.

Corresponding reference characters indicate corresponding parts throughout the several views. Although the drawings represent embodiments of the present invention, the drawings are not necessarily to scale and certain features may be exaggerated to better illustrate and explain the embodiments. The exemplifications set out herein illustrate embodiments of the invention in several forms and such exemplification is not to be construed as limiting the scope of the invention in any manner.

### DETAILED DESCRIPTION

The embodiments of the disclosure discussed below are not intended to be exhaustive or limit the invention to the precise forms disclosed in the following detailed description. Rather, the embodiments are chosen and described so that others skilled in the art may utilize their teachings.

Generally, in embodiments of a camera set forth herein, the camera includes an optical train and an image sensor having an imaging surface. The optical train includes two or more lenses aligned to form a line of sight of the camera. The optical train may also include a prism configured to guide the line of sight to the imaging surface. The optical train receives light reflected from an object in a target space, and the image sensor generates a corresponding electronic image or video for presentation with a display screen. The prism changes the orientation of the image stream reflected by the lenses to enable construction of a camera with reduced cross-sectional area. The electronic images are communicated to devices that may display the electronic images or may reformat the electronic images before they are displayed.

Since the intrusiveness of a medical procedure may be determined by the size of the camera, reducing the cross-sectional area of the camera may enable performance of comparably less intrusive medical procedures or performance of procedures in pediatric patients. Similarly, a smaller camera may also enable use of visualization instruments in spaces smaller than previously possible.

Embodiments of a visualization instrument including the aforementioned camera and others, and embodiments of a method of making the visualization instrument, are also disclosed herein. The visualization instrument is insertable into a space to capture images of tissues or objects located in the space. While the embodiments of the disclosure are applicable in medical and non-medical applications, exemplary features of visualization instruments will be described below with reference to a video laryngoscope. It should be understood that the invention is not so limited. The features described below may be equally applicable to any medical and non-medical applications and instruments.

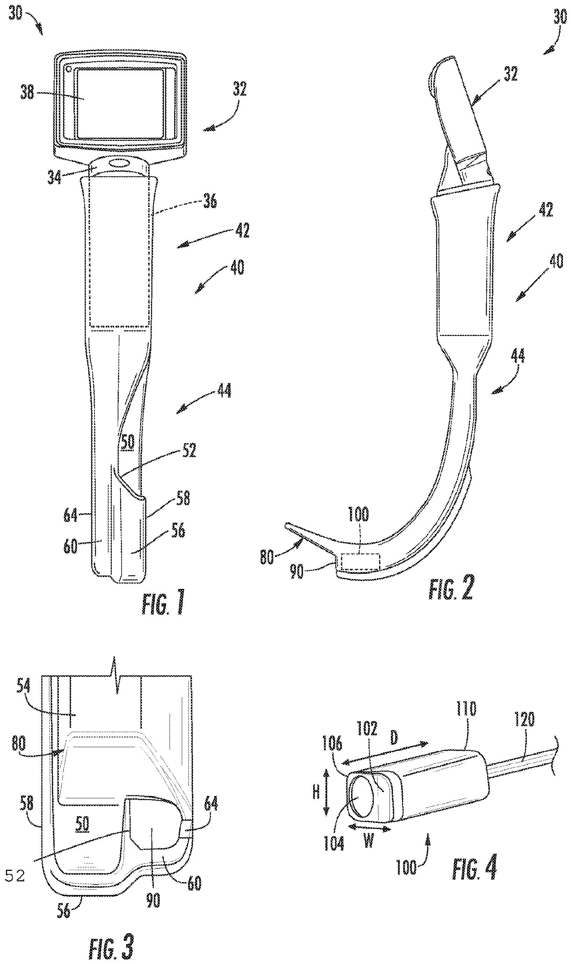

Referring to FIGS. 1, 2 and 3, an embodiment of a visualization instrument, illustratively video laryngoscope 30, comprises image presentation component 32 and a blade 40. Image presentation component 32 includes a display support structure 34, coupling a battery housing 36 and a display screen 38. Blade 40 includes a handle portion 42 integrally formed with an insertable portion 44. In the present embodiment, blade 40 is shown as a single part integrally combining handle portion 42 and insertable portion 44. In a variation thereof, the handle portion and the insertable portion are distinct parts that are removably attachable. Handle portion 42 comprises a proximal cavity configured to receive battery housing 36. When battery housing 36 is received in the proximal cavity, blade 40 supports image presentation component 32, forming a self-contained portable visualization instrument. A portable visualization instrument may be sized so that it can be hand-held. Blade 40 may be molded from polymeric materials.

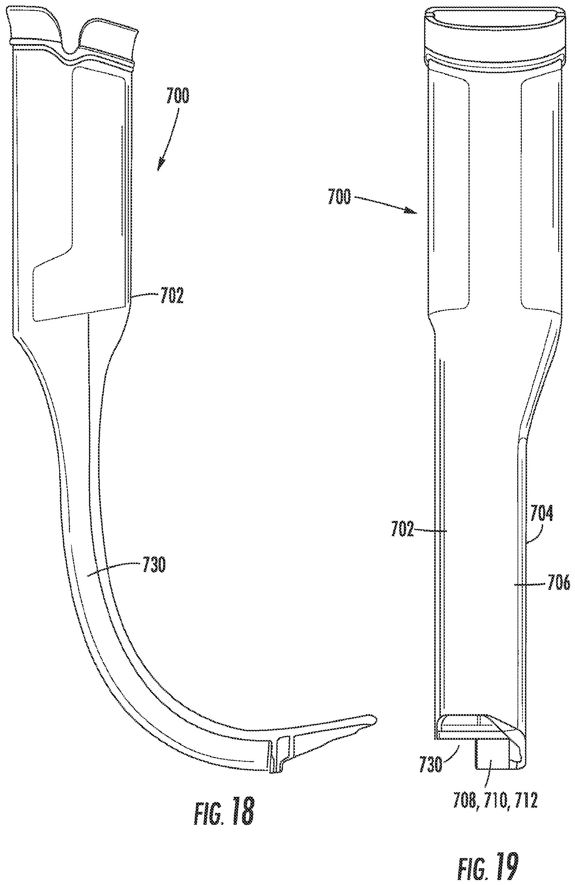

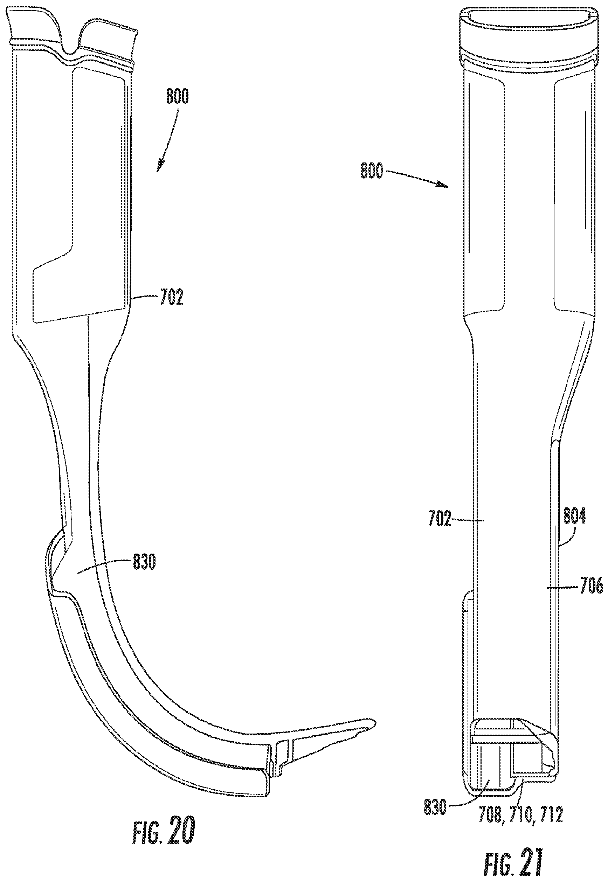

As shown, insertable portion 44 comprises an elongate guide pathway 50 configured to facilitate insertion of an endotracheal tube, catherer and the like (not shown) into the larynx of a patient. Guide pathway 50 is positioned on one side of a medial wall 52. Guide pathway 50 is further defined by an anterior guide wall 54, a posterior guide wall 56, and a lateral guide wall 58 (positioned opposite medial wall 52). An electronics pathway (not shown) is positioned on the opposite side of medial wall 52, in a side-by-side arrangement. The electronics pathway is defined by medial wall 52, anterior wall 54, a posterior electronics pathway wall 60 and a lateral electronics pathway wall 64. The cross-sectional area of the electronics pathway may have square, circular or any other shape. In a variation of the present embodiment, insertable portion 44 does not include lateral guide wall 58 or posterior guide wall 56, and the guide pathway is formed by the surfaces of medial wall 52 and anterior wall 54. The electronics pathway extends from a proximal end of insertable portion 44 to a blade view port 90 located at the distal end of insertable portion 44. A camera 100, shown in FIG. 4, is positioned at the distal end of the electronics pathway such that camera 100 illuminates the space in front of the distal end of insertable portion 44 (e.g. the target space) to capture images of tissues or an object positioned therein. In both variations of the present embodiment, anterior wall 54 extends beyond blade view port 90 to form a truncated tip 80, which is configured to lift the epiglottis. A translucent window may be sealably attached to cover blade view port 90 to seal the electronics pathway from moisture and dirt. An exemplary window is described with reference to FIGS. 19, 21 and 24-27. In a variation of the present embodiment, the blade is molded with an integral window such that a cover does not need to be sealably attached. In another variation of the present embodiment, the window is inserted in the mold of the blade, and the blade is molded around the periphery of the window to sealably attach the window. In any of the preceding variations, an anti-fog coating may added to the external surface of the cover.

Referring to FIG. 4, camera 100 comprises an LED cover 102, a support structure 106, an assembly housing 110 and a wire bundle 120. Exemplary wire bundles include flat cables, ribbon cables, a cable including the bundle of wires, and a bundle of individual wires, and any other configuration of wires. In embodiments herein, a camera harness comprises a camera, a wire bundle, a connecting member, and a sheath enclosing the wire bundle and forming therewith a camera harness. The camera harness is insertable into an electronics channel to position the camera adjacent the blade view port.

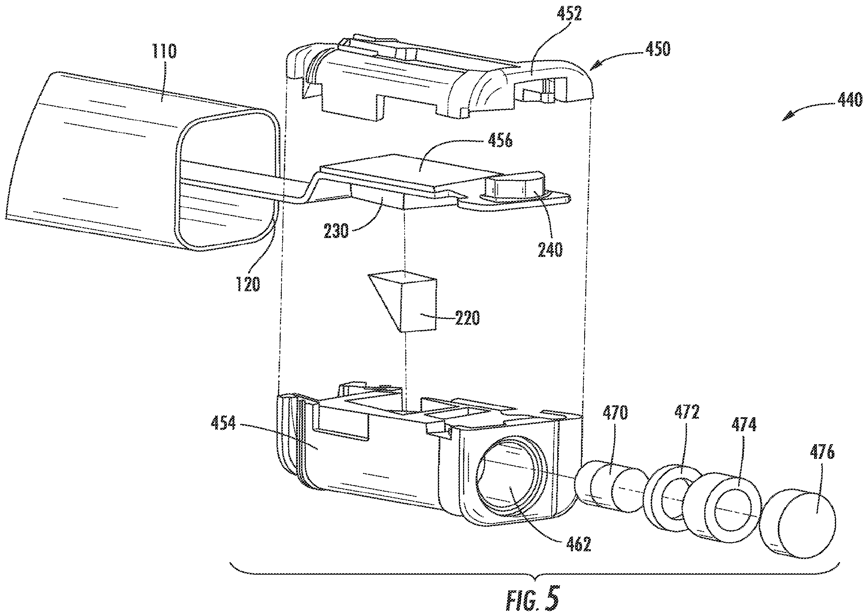

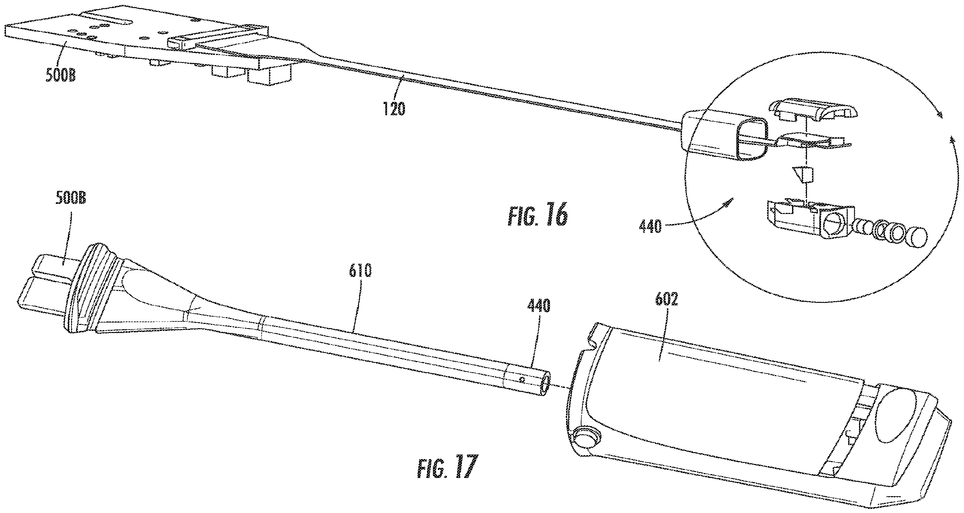

Camera 100 also comprises an optical train which may include a prism (show in FIGS. 5 and 16). Support structure 106 includes a camera view port 104 and is configured to support an LED and an image sensor. The LED illuminates the target space and the image sensor captures images of illuminated structures therein, such as tissues and objects. Wire bundle 120 is a conduit for the transfer of power, control signals and the video stream between the LED, the image sensor, and image presentation component 32. Assembly housing 110 encloses support structure 106. The LED and the image sensor may be potted within assembly housing 110. Assembly housing 110 may be made of a robust material to protect the LED and the image sensor. Exemplary robust materials include aluminium, copper, steel, and polymers, which may include glass or carbon fiber reinforcements. Assembly housing 110 may comprise a conductive metal to reduce electromagnetic interference.

The height, width and depth of camera 100 are denoted by the letters H, W and D. As used herein, the height and width of the cameras are intended to describe two substantially orthogonal dimensions of the cameras which do not necessarily correspond to the height and width of the electronics pathway. As shown in FIG. 4, the height of camera 100 corresponds to the anterior/posterior height of the blade, defined by the distance between the anterior and posterior guide walls, at the distal end of the blade. The width of the insertable portion includes the width of the guide pathway and the width of the electronics pathway, which is dimensioned to encompass the width of camera 100. In another variation (not shown), camera 100 is rotated relative to the orientation shown in FIG. 4, such that its width dimension is about parallel to the anterior/posterior height of the distal end of the blade. Camera 100 may be rotated relative to the anterior/posterior height by any amount.

The cameras present exemplary constructions configured to reduce the dimensions of the respective camera. The cameras may enable operation of multiple blade configurations with a common display support structure. With a common display support structure, multiple blades may be provided in a kit, such as an emergency response kit. Exemplary blades include adult and pediatric blades, and channelled and channelless blades (e.g. blades with and without posterior and lateral guide walls). A kit may also include a stylet, an endoscope and a snake-cam (a malleable camera harness with a camera) and any other device configured to operate with image presentation component 32.

By providing a power regulator in the camera, different LED configurations can be designed to satisfy different lighting requirements from the same power source. For example, one blade may receive a constant current from image presentation component 32, and another blade may convert the constant current to a different level of constant current suitable to provide a different light intensity or meet the rated requirements of a differently sized LED. In another example, a lamp is used instead of an LED, and the power regulator converts the power received from image presentation component 32 to a different level (higher or lower) or type (voltage to current or current to voltage). Further, the intensity of the LED may be controlled by a control signal from image presentation component 32 which changes a feedback loop coupled to the power regulator. For example, a feedback voltage or current may be changed by switching a transistor on or off to change a resistance in the feedback look, which resistance controls the output voltage or current of the power regulator (e.g. a switching regulator). Feedback loops used with power regulators are known.

An optional orientation processor may be provided. Use of an orientation processor facilitates use of different optical trains with a common image presentation component, without modification of the image presentation component. Alternatively, image presentation component may include video processing logic operable to re-orient the video stream if necessary. Re-orientation logic, power regulaors, and use of an orientation processor are more fully described in International Application No. PCT/US14/52780, filed August 26, 2014, which is incorporated herein in its entirety. The term "logic" as used herein includes software and/or firmware executing on one or more programmable processors, application-specific integrated circuits, field-programmable gate arrays, digital signal processors, hardwired logic, or combinations thereof. Therefore, in accordance with the embodiments, various logic may be implemented in any appropriate fashion and would remain in accordance with the embodiments herein disclosed. It should be understood that the electrical components described above, or functions performed by them, may be provided in image presentation component 32 or in the camera harness.

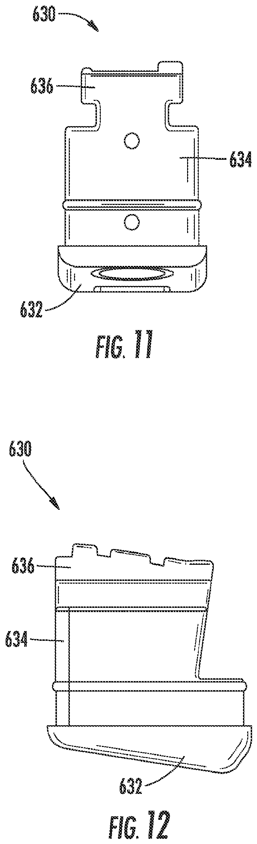

FIG. 5 is an exploded view of another embodiment of a camera, denoted by numeral 440. Camera 440 is similar to cameras 100 and 612 (described below) in respect to the optical train and inclusion of an image processor. Camera 440 includes a two-part support structure 450 comprising first support structure 454 and second support structure 452. First and second support structures 454 and 452 are adapted to enclose a prism 220, an image sensor 230, and an LED 240. Support structure 450 may also include a sheath engagement member, such as sheath engagement member 636 described with reference to FIGS. 11 and 12, to facilitate formation of a camera harness. As shown therein, polymeric material wraps around sheath engagement member 636 and connecting member 500B, so if the camera and the connector are pulled, the sheath prevents damage to wire harness 610.

Camera 440 further comprises lenses 470, 474, and 476, and a spacer 472, and may comprise a window (not shown). In one example, the window is sealingly attached to a view port 462 of first support structure 454 to seal the optical train from moisture and dirt. In another example, the window is omitted and an adhesive material is applied to the most distal lens, in this case lens 476, to seal the optical train. The adhesive material may comprise an ultraviolet curable optical clear adhesive. A power regulator may be provided to convert power received from image presentation component 32 to a different form or level. The power regulator may convert an input voltage to an output voltage of a different voltage value, or to a constant current, for example. The power regulator may be provided to power an orientation processor. The orientation processor may be provided to cause the image sensor to change the orientation of the video stream to compensate for the orientation changes due to the use of a prism. For example, the image processor may include an input pin which can be activated to select a particular orientation, and the orientation processor may be programmed to activate the selected pin. Re-orientation may be desired to compatibilize different blades and camera support structures with a common image presentation component 32. If compatibilization is not desired, the image stream may be reoriented by orientation logic in the image presentation component 32. Image presentation component 32 may convert the image stream output by image sensor 230 to a different size and transmit to resized image stream to a remote device.

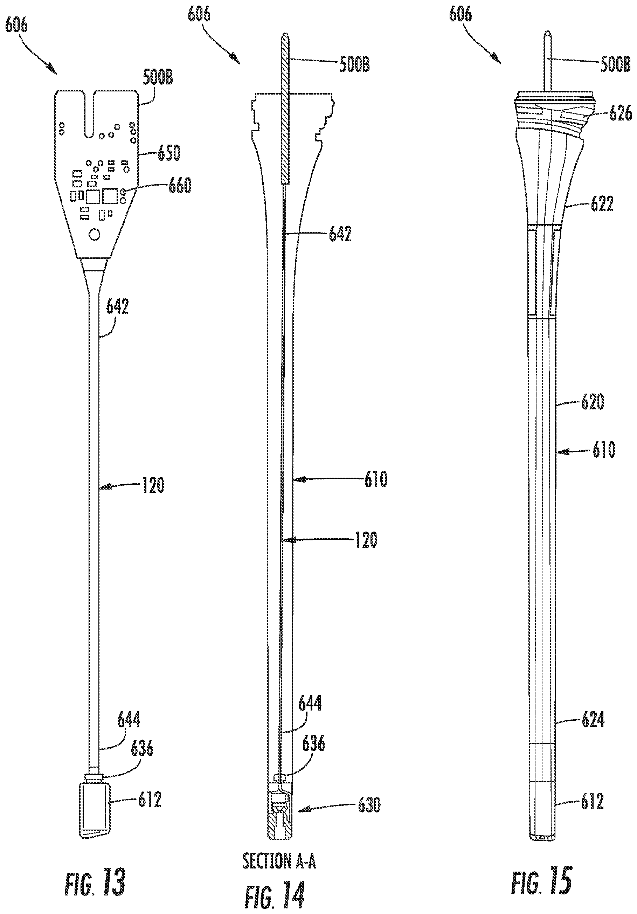

A stiffener component 456 may be coupled to wire bundle 120, illustratively a flexible flat cable, to ensure proper mounting of image sensor 230. Electronic components may be mounted on connecting member 500B, as shown in FIGS. 13 and 16 described further below. Small electronic components may be mounted on wire bundle 120. In one example, integrated circuits are mounted on connecting member 500B and resistors, capacitors, and other passive components are mounted on wire bundle 120. In one example, passive components are mounted opposite image sensor 230. LED 240 is supported by wire bundle 120. In the present embodiment, second support structure 452 includes a distal cavity 460 and LED 240 is positioned in distal cavity 460, but second support structure 452 does not fully enclose LED 240. In one variation, an adhesive material is applied to LED 240 and second support structure 452 to seal LED 240 in support structure 450. A stiffener such as stiffener 456 may also be attached along the length of wire bundle 120 to facilitate manufacturing of the camera harness.

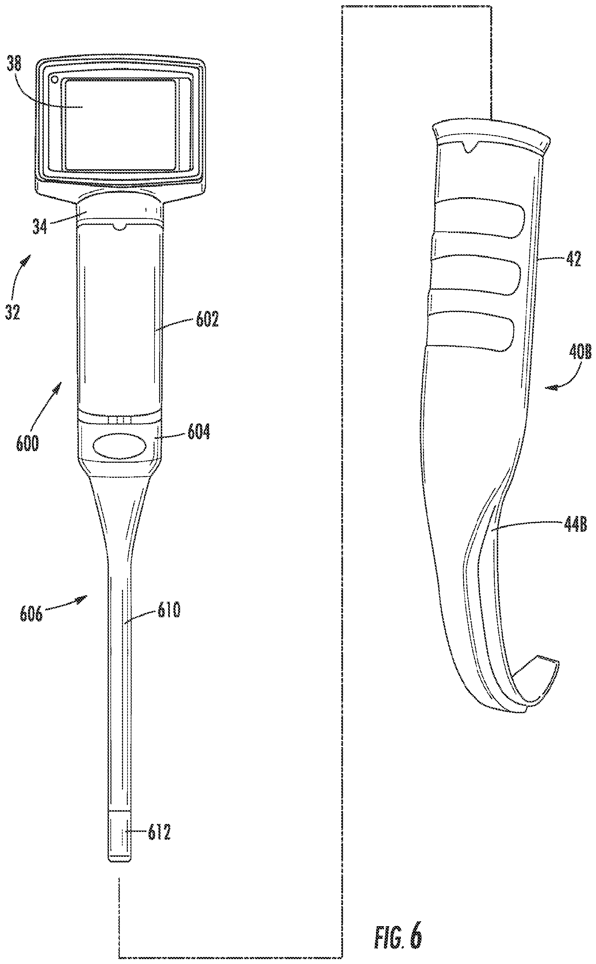

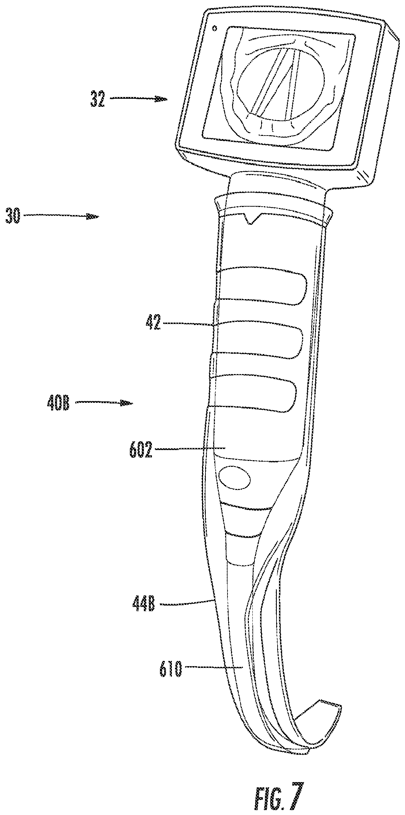

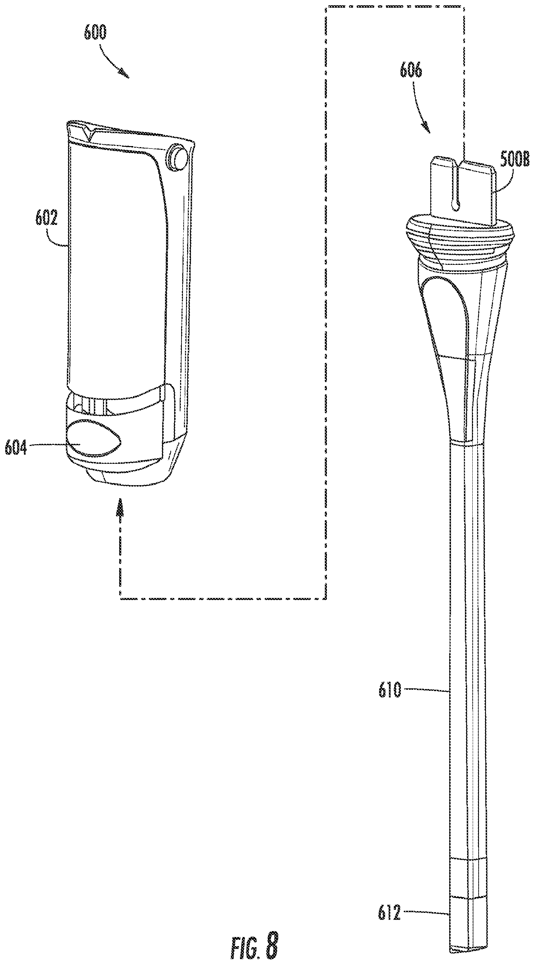

FIGS. 6 and 7 are exploded and assembled views of an embodiment of a video laryngoscope comprising image presentation component 32, a blade adapter 600 and a channelless blade 40B including an insertable portion 44B. FIG. 8 is an exploded view of blade adapter 600. Blade adapter 600 includes an adapter body 602, a sliding tab 604, a wire harness 610 and a camera 612. Wire harness 610 electrically couples image presentation component 32 to camera 612. Camera 612 is similar to the cameras described herein. Adapter body 602 includes a proximal cavity (not shown) configured to receive battery housing 36. Adapter body 602 is attached to battery housing 36 by sliding tab 604 from an unlocked position to a locked position. Once locked, blade adapter 600 remains attached to image presentation component 32. Wire harness 610 comprises a sheath that encloses wire bundle 120, which is permanently connected to camera 612. When adapter body 602 receives battery housing 36, camera harness 606 is received by the electronics pathway such that the camera view port is adjacent the blade window. Blade adapter 600 also comprises a connecting member 500B (FIG. 8) located in the proximal cavity suitable to electrically couple camera harness 606 to image presentation component 32.

Adapter body 602 is well suited to securely attach camera harness 606 to image presentation component 32 by enclosing battery housing 36 and enabling a blade to enclose camera harness 606 and adapter body 602. In some embodiments, image presentation component 32 is attached to camera harness 606 without use of adapter body 602. For example, a blade may enclose battery housing 36 and camera harness 606 without use of an adapter body. Advantageously, adapter bodies enable use of commonly sized blades with differently sized battery housings. Alternatively, adapter bodies enable use of commonly sized battery housing with differently sized blades.

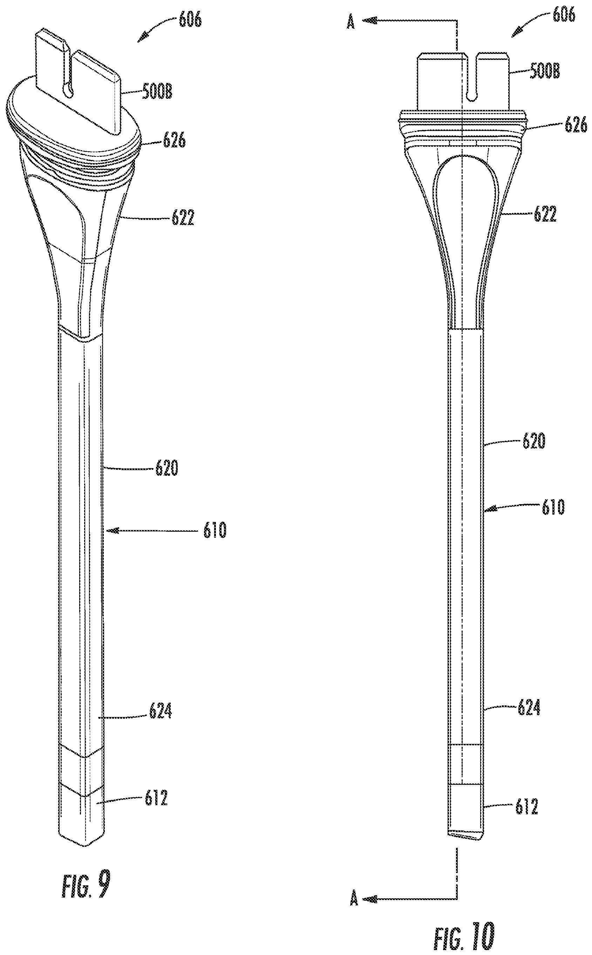

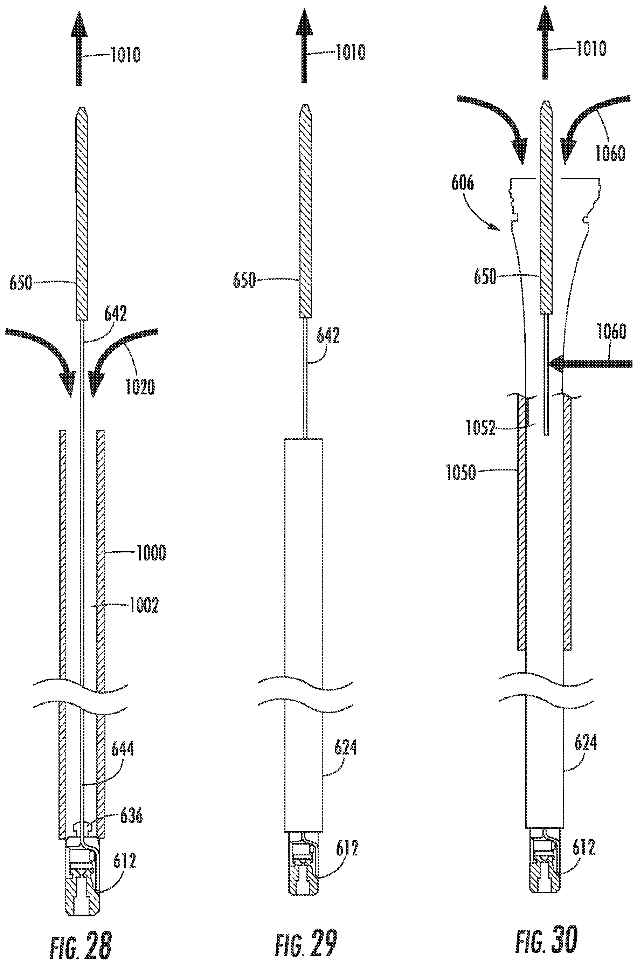

FIGS. 9 and 10 are perspective and plan views of camera harness 606. Camera harness 606 comprises wire harness 610 which includes a sheath 620 enclosing wire bundle 120 (not shown) and having a camera end sheath 624 opposite a proximal end sheath 622. A coupling member 626 is disposed on proximal end sheath 622, which is configured to couple with a complementary coupling member (not shown) disposed in adapter body 602 once camera harness 606 slides through the proximal cavity therein, as indicated by the dashed arrow in FIG. 8. In one embodiment, sheath 620 is injection molded over wire bundle 120 to form wire harness 610. Camera end sheath 624 may be injection molded in one shot, and proximal end sheath 622 may be injection molded in a second shot. The second shot may reach camera end sheath 624 and thermally bond therewith. A novel embodiment of a two-shot injection molding method is described below with reference to FIGS. 28-30. In another embodiment, sheath 624 is liquid cast in one shot, in a process well known in the art. Although liquid casting materials may be more expensive, liquid casting has the advantage of being a cold forming process, also referred to as a cold molding process, and is therefore more easily controllable.

FIGS. 11 and 12 are plan views of an embodiment of a support structure of camera 612, illustratively support structure 630. Support structure 630 functions similarly to support structure 106, in that it supports the optical train, the LED and the image sensor. Support structure 630 includes a distal wall 632, a body 634 and a sheath engagement member 636. Camera end sheath 624 is formed over sheath engagement member 636 to mechanically secure camera 612 to wire harness 610. In one example, camera end sheath 624 is injection molded over the wire bundle at a temperature sufficiently high to form a thermal bond with sheath engagement member 636. Assembly housing 110 slides over body 634 until it contacts distal wall 632. The polymeric material of which sheath 620 is formed wraps around sheath engagement member 636 and connecting member 500B, so if the camera and the connector are pulled, sheath 620 prevents damage to wire harness 610 by the pulling tension.

FIGS. 13 to 15 are plan and cross-sectional views of camera harness 606. FIG. 13 illustrates a circuit board 650 including connecting member 500B and a plurality of electronic components 660. Exemplary electronic components include passive and active components. Exemplary active components include power regulators and microcontrollers or processors. Exemplary passive components include capacitors and resistors. FIG. 14 is a cross-sectional view of camera harness 606 showing wire bundle 120 connecting circuit board 650 and camera 612. Wire bundle 120 includes a camera end 644 and a proximal end 642.

FIGS. 16 and 17 are perspective views of another embodiment of an adapter, similar to adapter 600. In the present embodiment, the adapter includes adapter body 602, and wire harness 610 enclosing wire bundle 120, which is permanently connected to camera 440, previously described with reference to FIG. 5. Wire bundle 120, illustratively a flexible flat cable, extends from camera 440 to a circuit board including connecting member 500B, where it is attached to the circuit board with a mechanical brace. As shown, electronic components such as a power regulator, an orientation processor, and other active or passive components may be located on the circuit board, as described with reference to FIG. 13. Removal of these components from the camera is desirable to achieve camera size reductions.

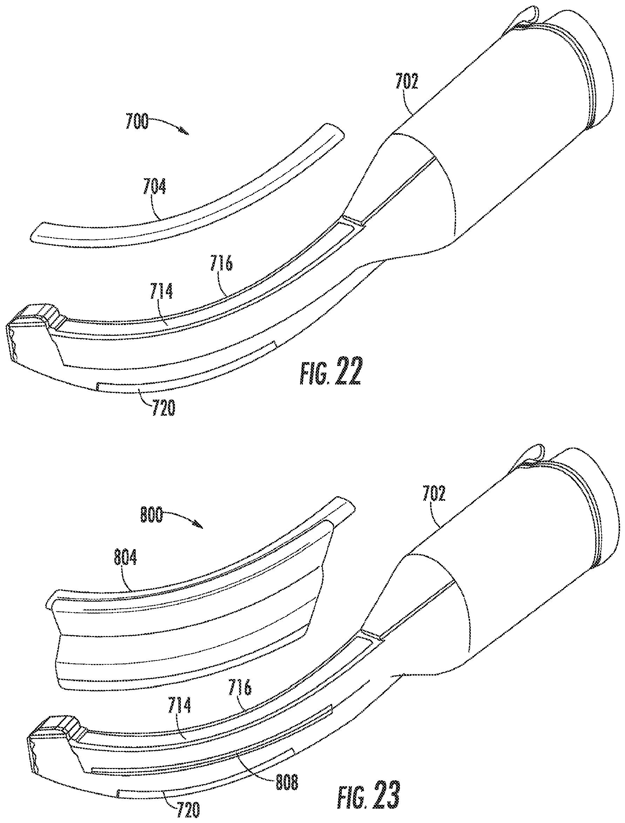

FIGS. 18 to 23 are plan and exploded views of embodiments of channelled and channelless blades, illustratively blades 700 and 800. In the present embodiment, blades 700 and 800 include a blade body 702, a blade view port 708, a window 710, and optionally, a window anti-fog coating 712. Window 710 is sealingly secured to blade view port 708. In another embodiment, a window is integrally formed with the blade, as described previously. Blade 700 is channelless and includes a guide pathway 730 formed by an anterior and a medial wall. An affixable wall 704 encloses an electronics channel 714 (best seen in FIG. 22) formed by blade body 704. A wall 716 is shown to better illustrate electronics channel 714. Wall 716 is the anterior wall of the blade. Electronics channel 714 is formed by an anterior wall, the medial wall, a posterior wall, and an affixable wall, illustratively wall 704, opposite the medial wall. As defined herein, the anterior side is opposite the posterior side, which is the side on which the display screen is located. If a patient is laying face-up, and the visualization instrument is inserted into the patient, the anterior side will face up so that its tip can contact the epiglottis, and the posterior side will face down. Affixable wall 704 is affixed to blade body 702 by a seam 706. Exemplary seams include adhesive and thermal seams. A thermal seam may be formed by ultrasonically welding blade body 702 and wall 704. Advantageously, a blade formed from two parts enables forming the body, which is the more complex part, by injection molding. Therefore, the blade can be manufactured at a cost low enough that the blade can be discarded after each use.

In another embodiment, wall 704 may include a portion of the anterior wall or a portion of the posterior wall, thus having an "L" shaped cross-section, or portions of both, thus having a "U" shaped cross-section. In a further embodiment, the affixable wall may comprise the anterior or posterior walls of the electronics channel, with or without portions of the lateral wall.

Blade 800 is channelled and includes a guide pathway 830 formed by anterior, medial, posterior and lateral walls. A wall 804 encloses an electronics channel 714 (best seen in FIG. 23). Wall 804 is affixed to body 702 by a seam 706. A secondary seam 808 may be provided to further support wall 804. Wall 804 is similar to wall 704 but further includes the posterior walls of electronics channel 714 and guide pathway 830, and the lateral walls of guide pathway 830, one of which extends from the medial wall and the other being an outer wall. Blades 700 and 800 may include a ridge 720 extending posteriorly from the anterior wall which assists in the guiding function performed by the guide pathways. Exemplary seams include adhesive seams, thermal seams, and interference fit seams. The seams may be sealing seams, wherein a seal is formed where the wall contacts the body to form the seam. If the camera end of the electronics channel is also sealed, for example by integral formation or affixation of the window, the portion of the electronics channel coextensive with the insertable portion of the blade will be sealed to prevent moisture of the patient from entering the electronics channel. Of course the portion of the electronics channel adjacent the handle of the blade is open and not sealed, at least not until presentation component 32 is coupled to the cavity in the handle.

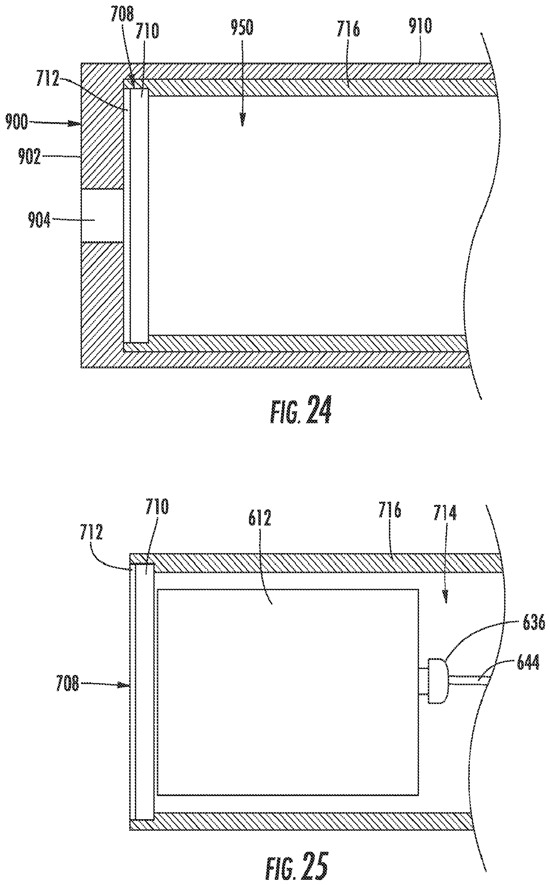

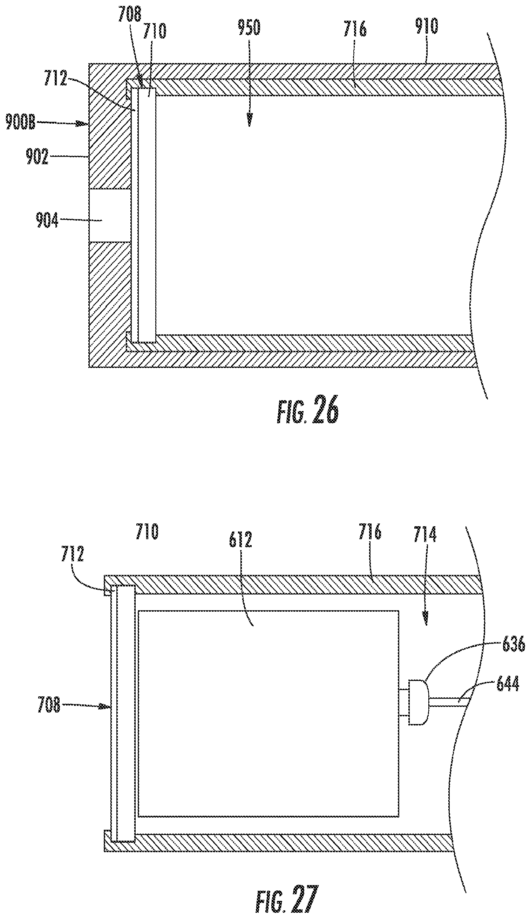

FIGS. 24 and 26 are partial views of embodiments of an injection molding mold, illustratively molds 900 and 900B, for forming a blade body and securing the blade window in one shot. FIGS. 25 and 27 are partial views of embodiments of blades formed in molds 900 and 900B with camera 612 positioned in electronics channel 714. Mold 900 includes a frame having a distal wall 902 having a vacuum port 904 therein, and lateral walls 910 forming an injection molding cavity. Blade window 710, having optionally anti-fog coating 712, is secured to distal wall 902 by vacuum. A sliding mold 950 exerts pressure against blade window 710 to prevent molten polymer injected into the molding cavity from covering window 710 except where desired. A shot of polymer is injected into the molding cavity under pressure to fill a gap between sliding mold 950 and lateral walls 910. The shot forms wall 716. A portion of the shot contacts the proximal surface of blade window 710 and another portion contacts the peripheral surface of blade window 710. The polymer temperature and pressure are set to generate a shot that is sufficiently hot to thermally bond blade window 710. After the shot, sliding mold 950 is held in position until blade window 710 and wall 716 are thermally bonded. Sliding mold 950 is the removed from the molding cavity. Then, the blade is removed from the molding cavity with the blade window sealingly secured to blade view port 708. Mold 900B is similar to mold 900 except that it includes a groove on the inside surface of distal wall 902. When the shot is injected into the molding cavity, the polymer flows into the groove, thereby forming a channel on wall 716 that mechanically retains blade window 710 and anti-fog coating 712. Of course, a thermal bond may also be formed to seal blade window 710 in blade view port 708. Thermal bonding of blade window 710 to the blade's body is beneficial when the anti-fog coating prevents adhesively bonding blade window 710 to blade view port 708.

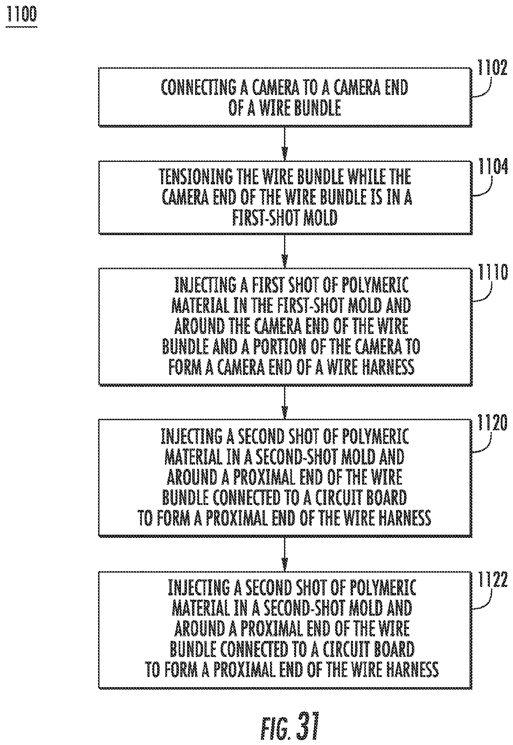

FIGS. 28-30 are cross-sectional views of molds used in an embodiment of a two-shot molding process described with reference to FIG. 31. A first-shot mold 1000, having a first-shot mold cavity 1002, and a second-shot mold 1050, having a second-shot mold cavity 1052, are used. Arrows 1010 indicate tension applied to wire bundle 120. Arrows 1020 indicate injection of polymeric material in a first shot and arrows 1060 indicate injection of polymeric material in a second shot. The polymeric material in the first shot flows in first shot mold cavity 1002 until it encloses sheath engagement member 636. First shot mold 1000 is then replaced by second-shot mold 1050 and a second shot of polymeric material is injected into second-shot mold cavity 1052. Although not shown, second-shot mold 1050 may extend to enclose a portion of circuit board 650. Second shot mold 1050 is then removed from newly formed camera harness 606. In one variation of the present embodiment, a lubricating material is applied to the wire bundle to facilitate the flow of the polymeric material around the wire bundle without stressing or deforming the wire bundle. In one example, the lubricating material comprises curable silicone and is applied before injection of the first shot. The lubricating material may be applied to the wire bundle prior to attachment to the camera or thereafter. The curable silicone may be heat curable and may be cured before or during the first shot. The curable silicone may be cross-linked in any other manner, such as by ultraviolet treatment, catalyzation, or aging. The curable silicone may be referred to as a "conformal coating." Advantageously, the conformal coating does not leach or migrate to the surface of the sheath. Any non-migratory coating may be applied instead to lubricate the wire bundle and reduce friction with the molten polymeric material. The lubricating material may be sprayed, spread or coated. The wire bundle may also be dipped in the lubricating material.

Referring to a flowchart 1100 illustrated in FIG. 31, an embodiment of a two shot injection molding method to form a camera harness will now be described. The method begins at 1102, by connecting a camera to a camera end of a wire bundle. Before or after said connecting, the wire bundle may be coated with a lubricating material. Exemplary lubricating materials include silicone, curable silicone, and surfactants.

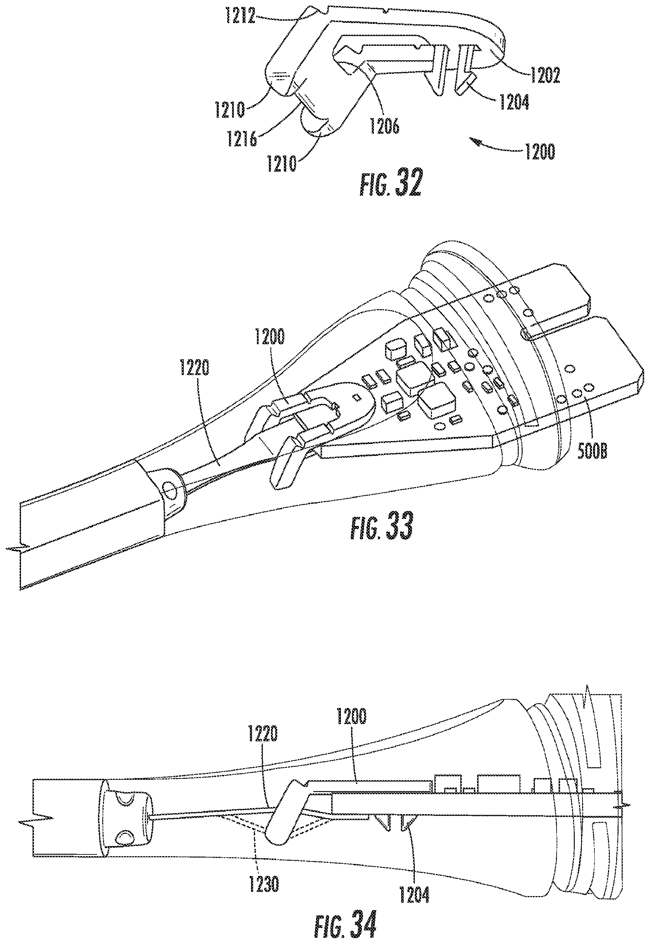

The method continues at 1104, by tensioning the wire bundle while the camera end of the wire bundle is in a first-shot mold. Additionally, the wire bundle may be centered in the first-shot mold. In one example, centering is performed using a positioning device described in FIGS. 32-34.

The method continues at 1110, by injecting a first shot of polymeric material in the first-shot mold and around the camera end of the wire bundle and a portion of the camera to form a camera end of a wire harness. If curable silicone is used, the curable silicone is cured before injecting the first-shot. The camera end of the wire harness may be removed from the first-shot mold.

The method continues at 1120, by injecting a second shot of polymeric material in a second-shot mold and around a proximal end of the wire bundle connected to a circuit board to form a proximal end of the wire harness.

The method continues at 1122, by removing the distal end of the wire harness from the second-shot mold.

FIGS 32-34 illustrate a positioning device 1200. Referring to FIG. 32, positioning device 1200 comprises a contact surface 1202 adapted to attach to a circuit board including connecting member 500B. Tensioning device 1200 further comprises positioning surfaces 1206 and 1216, and guiding portions 1210 and 1212. Positioning surfaces 1206 and 1216 are on a different plane than contact surface 1202. Referring to FIG. 34, in one path, numbered 1120, the wire bundle contacts surface 1206 between guiding portions 1212. In another path, numbered 1230, the wire bundle contacts surface 1216 between guiding portions 1210. The wire bundle and positioning device 1200 are attached to the circuit board and the wire bundle is extended from the circuit board between guiding portions 1210 until it contacts positioning surface 1216. Alternatively, the wire bundle is extended from the circuit board between guiding portions 1212 until it contacts positioning surface 1206, providing a different placement of the wiring bundle relative to the second-shot mold. Positioning device 1200 in conjunction with the circuit board may also eliminate movement or vibration of the wiring bundle during injection molding by applying pressure on two spaced apart opposing surfaces of the wire bundle, forming an S-wrap, when tension is applied during the injection molding process. Other variations of the foregoing embodiment may also be provided. More generally, a centering device includes two surfaces configured to contact the wire bundle on two spaced apart opposing surfaces of the wire bundle.

While the invention has been described as having exemplary designs, the present disclosure may be further modified within the scope of this disclosure. This application is therefore intended to cover any variations, uses, or adaptations of the disclosure using its general principles. Furthermore, this application is intended to cover such departures from the present disclosure as come within known or customary practice in the art to which this invention pertains.

## Claims

1. A visualization instrument comprising:
an image presentation component (32) including a display screen (38) and a battery housing (26);
a camera harness (606) coupled to the image presentation component (32), the camera harness (606) including a wire harness (610) and a camera (100, 450, 612), the camera including a light source (240) configured to illuminate structures in a target space, an image sensor (230), and an optical train including one or more lenses (470, 474), the optical train configured to receive light reflected from the illuminated structures and refract optical images of the illuminated structures to the image sensor (230), the image sensor (230) generating an image stream corresponding to the optical images;
a blade (40) comprising a blade body (702) including a handle portion (42) integrally formed with an insertable portion (44), the handle portion (42) having a proximal cavity configured to receive the battery housing (26), the insertable portion (44) comprising a guide pathway (50) adapted to guide an elongate device, **characterised in that** the blade further comprises an electronics pathway formed by the blade body (702) and an affixable wall (704) seamed to the body portion to form a sealed seam, and a window (710) at a camera end of the blade body (702), wherein the camera end of the blade body (702) surrounds and contacts the periphery of the window (710), the electronics pathway (50) configured to receive the camera harness (606) when the image presentation component (32) is inserted into the proximal cavity of the blade (40), with the camera (100, 450, 612) positioned adjacent the window (710).

2. A visualization instrument as in claim 1, wherein the camera end of the blade body (702) is formed around the periphery of the window (710).

3. A visualization instrument as in claim 1, wherein the camera end of the blade body (702) is integrally formed with the window.

4. A visualization instrument as in any one of claims 1, 2 or 3, wherein the wire harness (610) comprises a wire bundle (120), a cured silicon layer on the wire bundle (120), and a polymeric sheath (620) enclosing the wire bundle (120) and the cured silicon layer.

5. A visualization instrument as in any one of claims 1-4, wherein the wire harness (610) comprises a wire bundle (120), a polymeric sheath (620) enclosing the wire bundle (120), and a circuit board (650) connected to the wire bundle opposite the camera (100, 450, 612), further comprising a blade adapter body (602) intermediate battery housing (36) and handle portion (42), the blade adapter body (602) connected to the wire harness (610) and having an adapter cavity through which the circuit board (650) protrudes to mate with a corresponding electronic connector in the image presentation component (32).

6. A visualization instrument as in claim 5, the adapter body (602) attached to the battery housing (36) and locked in place by a sliding tab (604).

7. A visualization instrument as in any one of the preceding claims, wherein the blade (40) comprises a curvature along a longitudinal direction thereof.

## Patentansprüche

1. Visualisierungsinstrument, umfassend:
eine Bilddarstellungskomponente (32) mit einem Anzeigebildschirm (38) und einem Batteriegehäuse (26);
einen mit der Bilddarstellungskomponente (32) gekoppelten Kamerastrang (606), wobei der Kamerastrang (606) einen Kabelstrang (610) und eine Kamera (100, 450, 612) umfasst, wobei die Kamera eine Lichtquelle (240), die ausgestaltet ist, Strukturen in einem Zielraum zu beleuchten, einen Bildsensor (230) und eine optische Anordnung mit einer oder mehreren Linsen (470, 474) umfasst, wobei die optische Anordnung ausgestaltet ist, von den beleuchteten Strukturen reflektiertes Licht aufzunehmen und optische Bilder der beleuchteten Strukturen zu dem Bildsensor (230) abzulenken, wobei der Bildsensor (230) entsprechend den optischen Bildern einen Bildstrom erzeugt;
einen Spatel (40), umfassend einen Spatelkörper (702) mit einem Griffteil (42), der integral mit einem einführbaren Teil (44) ausgebildet ist, wobei der Griffteil (42) einen proximalen Hohlraum aufweist, der ausgestaltet ist, das Batteriegehäuse (26) aufzunehmen, wobei der einführbare Teil (44) eine Führungsbahn (50) umfasst, die ausgelegt ist, eine längliche Vorrichtung zu führen, **dadurch gekennzeichnet, dass** der Spatel ferner eine Elektronikbahn, die durch den Spatelkörper (702) und eine befestigbare Wand (704) ausgebildet ist, die an den Körperteil gefalzt ist, um einen abgedichteten Falz auszubilden, und ein Fenster (710) an einem Kameraende des Spatelkörpers (702) umfasst, wobei das Kameraende des Spatelkörpers (702) den Umfang des Fensters (710) umgibt und kontaktiert, wobei die Elektronikbahn (50) ausgestaltet ist, den Kamerastrang (606) aufzunehmen, wenn die Bilddarstellungskomponente (32) in den proximalen Hohlraum des Spatels (40) eingeführt ist, wobei die Kamera (100, 450, 612) angrenzend an das Fenster (710) positioniert ist.

2. Visualisierungsinstrument nach Anspruch 1, wobei das Kameraende des Spatelkörpers (702) um den Umfang des Fensters (710) ausgebildet ist.

3. Visualisierungsinstrument nach Anspruch 1, wobei das Kameraende des Spatelkörpers (702) mit dem Fenster integral ausgebildet ist.

4. Visualisierungsinstrument nach einem der Ansprüche 1, 2 oder 3, wobei der Kabelstrang (610) ein Drahtbündel (120), eine ausgehärtete Siliziumschicht an dem Drahtbündel (120) und eine Polymerhülle (620) umfasst, die das Drahtbündel (120) und die ausgehärtete Siliziumschicht umschließt.

5. Visualisierungsinstrument nach einem der Ansprüche 1-4, wobei der Kabelstrang (610) ein Drahtbündel (120), eine das Drahtbündel (120) umschließende Polymerhülle (620) und eine gegenüber der Kamera (100, 450, 612) mit dem Drahtbündel verbundene Leiterplatte (650) umfasst, ferner umfassend einen Spateladapterkörper (602) zwischen dem Batteriegehäuse (36) und dem Griffteil (42), wobei der Spateladapterkörper (602) mit dem Kabelstrang (610) verbunden ist und einen Adapterhohlraum aufweist, durch den die Leiterplatte (650) hervorsteht, um mit einem entsprechenden elektronischen Verbinder in der Bilddarstellungskomponente (32) in Eingriff zu kommen.

6. Visualisierungsinstrument nach Anspruch 5, wobei der Adapterkörper (602) an dem Batteriegehäuse (36) angebracht und durch eine Schiebelasche (604) festgestellt ist.

7. Visualisierungsinstrument nach einem der vorhergehenden Ansprüche, wobei der Spatel (40) eine Krümmung entlang einer Längsrichtung davon aufweist.

## Revendications

1. Instrument de visualisation, comprenant :
un composant de présentation d'image (32) incluant un écran d'affichage (38) et un boîtier de batterie (26) ;
un câblage de caméra (606) couplé au composant de présentation d'image (32), le câblage de caméra (606) incluant un câblage électrique (610) et une caméra (100, 450, 612), la caméra incluant une source de lumière (240) configurée pour éclairer des structures dans un espace cible, un capteur d'image (230), et un train optique incluant une ou plusieurs lentilles (470, 474), le train optique étant configuré pour recevoir de la lumière réfléchie à partir des structures éclairées et réfracter des images optiques des structures éclairées vers le capteur d'image (230), le capteur d'image (230) générant un flux d'images correspondant aux images optiques ;
une lame (40) comprenant un corps de lame (702) incluant une partie poignée (42) formée de façon monobloc avec une partie insérable (44), la partie poignée (42) ayant une cavité proximale configurée pour recevoir le boîtier de batterie (26), la partie insérable (44) comprenant un chemin de guidage (50) adapté pour guider un dispositif allongé, **caractérisé en ce que** la lame comprend en outre un chemin d'électronique formé par le corps de lame (702) et une paroi fixable (704) assemblée à la partie corps pour former un assemblage étanche, et une fenêtre (710) à une extrémité caméra du corps de lame (702), dans lequel l'extrémité caméra du corps de lame (702) entoure et entre en contact avec la périphérie de la fenêtre (710), le chemin d'électronique (50) étant configuré pour recevoir le câblage de caméra (606) lorsque le composant de présentation d'image (32) est inséré dans la cavité proximale de la lame (40), avec la caméra (100, 450, 612) positionnée de façon adjacente à la fenêtre (710).

2. Instrument de visualisation selon la revendication 1, dans lequel l'extrémité caméra du corps de lame (702) est formée autour de la périphérie de la fenêtre (710).

3. Instrument de visualisation selon la revendication 1, dans lequel l'extrémité caméra du corps de lame (702) est formée de façon monobloc avec la fenêtre.

4. Instrument de visualisation selon l'une quelconque des revendications 1, 2 ou 3, dans lequel le câblage électrique (610) comprend un faisceau de conducteurs (120), une couche de silicium durcie sur le faisceau de conducteurs (120), et une gaine polymérique (620) entourant le faisceau de conducteurs (120) et la couche de silicium durcie.

5. Instrument de visualisation selon l'une quelconque des revendications 1 à 4, dans lequel le câblage électrique (610) comprend un faisceau de conducteurs (120), une gaine polymérique (620) entourant le faisceau de conducteurs (120), et une carte de circuit imprimé (650) connectée au faisceau de conducteurs de façon opposée à la caméra (100, 450, 612), comprenant en outre un corps adaptateur de lame (602) entre le boîtier de batterie (36) et la partie poignée (42), le corps adaptateur de lame (602) étant connecté au câblage électrique (610) et ayant une cavité d'adaptateur à travers laquelle la carte de circuit imprimé (650) fait saillie pour s'accoupler à un connecteur électronique correspondant dans le composant de présentation d'image (32).

6. Instrument de visualisation selon la revendication 5, le corps adaptateur (602) étant attaché au boîtier de batterie (36) et verrouillé en place par une languette coulissante (604).

7. Instrument de visualisation selon l'une quelconque des revendications précédentes, dans lequel la lame (40) comprend une courbure le long d'une direction longitudinale de celle-ci.
